# EUROPEAN PATENT APPLICATION

(11) **EP 2 848 935 A1**
(43) Date of publication of application: **18.03.2015**
(21) Application number: 13788597.6
(22) Date of filing: 09.05.2013
(51) Int. Cl.: G01N 33/48, G01N 1/10

(54) **PROCESS FOR REMOVING RED BLOOD CELLS AND CENTRIFUGAL TUBE FOR BLOOD COLLECTION**

(30) Priority: 10.05.2012 JP 2012108420
(71) Applicant: Konica Minolta, Inc., Tokyo 100-7015 (JP)
(72) Inventor: CHIYODA, Tsuneko, Tokyo 100-7015 (JP); MIYAZAKI, Koji, Tokyo 100-7015 (JP); ARAKI, Jungo, Tokyo 100-7015 (JP)
(74) Representative: Gille Hrabal
(86) International application number: PCT/JP2013/063041
(87) International publication number: WO 2013/168767

(57) **Abstract**

It is an object of the present invention to provide a method of removing red blood cells and a centrifuge tube for blood collection, which method and centrifuge tube can, when cells contained in blood are observed after spreading the cells, improve detection accuracy of rare cells contained in the blood, hardly causing loss of the rare cells, even with a blood-derived sample stored for a long period-for example, several days-after blood collection until the separation of red blood cells. The method of removing red blood cells is a method for preliminarily removing red blood cells contained in a blood-derived sample for detection of a rare cell(s) potentially contained in the blood-derived sample, which method includes the steps of: (A) fixing cells contained in a blood-derived sample; and (B) subjecting the blood-derived sample obtained in the Step (A) to density gradient centrifugation to separate the sample into at least two layers, one of which contains a large amount of red blood cells and the other of which contains a large amount of cells other than red blood cells.

## Description

### TECHNICAL FIELD

The present invention relates to a method for preliminarily removing red blood cells contained in a large amount in a blood-derived sample, for detection of a rare cell (s) potentially contained in the blood-derived sample, and a centrifuge tube for blood collection in which a cell fixative, an anticoagulant and a separation liquid for density gradient centrifugation are placed.

### BACKGROUND ART

Circulating tumor cells (CTCs), vascular endothelial cells, vascular endothelial progenitor cells, various stem cells and the like (collectively referred to as "rare cells" in the present description) are cells that are extremely rarely present in peripheral blood depending on pathological conditions. Although detection of such rare cells is clinically obviously useful, detection of all rare cells in a whole-blood sample is still difficult in most cases.

In recent years, detection of rare cells has been attempted by application of various cell separation methods, and products for such methods are commercially available. It has been thought, in all of these cases, that effectiveness of detection results and means for securing the effectiveness are important because of rarity and heterogeneity of the cells to be detected. For example, rare cells may be lost after several steps of cell separation, or identification of rare cells may be difficult due to contamination with various unnecessary cells. It is therefore important to evaluate how much the rare cells are lost and to prevent loss of the rare cells.

For example, Patent Document 1 discloses a method in which blood is collected from a cancer patient into a Cyto-Chex (registered trademark) stabilizer, and subjected to normal centrifugation for removal of plasma, followed by using magnetic particles having an antibody specific to a CTC surface antigen immobilized thereon, to perform magnetic separation of CTCs.

However, CTCs are derived from metastatic breast cancer, prostate cancer, colon cancer and the like, and do not necessarily have the same properties (for example, even CTCs derived from the same tissue of colon cancer do not necessarily have the same types and numbers of molecules expressed on the cell surface (surface antigens)). Therefore, certain types of CTCs might be missed in the process of magnetic separation in Patent Document 1.

Patent Document 2 discloses a method for detecting rare cells in blood, which method comprises: a pretreatment step of removing red blood cells from blood to obtain a cell suspension containing leukocytes and rare cells; a cell spreading step of spreading the cell suspension in an observation area where a plurality of holes are formed; a photographing step of optical photographing of the cells spread in the observation area; and a detection step of detecting rare cells in the image obtained by photographing. This document describes that red blood cells may be separated by density gradient centrifugation in the pretreatment step (Paragraph [0038]), and that, in cases where the cells are fixed by the formalin method or the like, the fixation is carried out after centrifugation (when the cells are spread in the cell observation member, or at the same time as staining treatment for observation of the cells) (Paragraphs [0042] and [0043]).

However, in the detection method of Patent Document 2, a long time is required after blood collection to carry out the detection method. It is therefore thought that the cells in the blood are likely to be deteriorated. In particular, in cases where density gradient centrifugation is carried out in the pretreatment step, the recovery of rare cells is low, or the mononuclear cell layer is contaminated with a large amount of red blood cells. This inhibits the processes after the pretreatment step, and accurate results cannot be obtained in some cases.

### CITATION LIST

### [Patent Documents]

[Patent Document 1] Japanese Unexamined Patent Application Publication (Translation of PCT Application) No. 2005-501236
[Patent Document 2] WO2011/108454

### SUMMARY OF THE INVENTION

### PROBLEMS TO BE SOLVED BY THE INVENTION

It is an object of the present invention to provide a method of removing red blood cells and a centrifuge tube for blood collection, which method and centrifuge tube can, when cells contained in blood are observed after spreading the cells, improve detection accuracy of rare cells contained in the blood, hardly causing loss of the rare cells, even with a blood-derived sample stored for a long period-for example, several days-after blood collection until the separation of red blood cells.

### MEANS FOR SOLVING THE PROBLEMS

Usually, cell fixation using formaldehyde or the like causes intramolecular or intermolecular cross-linking of proteins and the like contained in the cells. In some cases, cells which have been fixed may keep almost the same specific gravity even after the fixation, but in other cases, the specific gravity may change to a lesser or greater extent irrespective of the type of the cells. However, since changes in the specific gravity after cell fixation have not been studied so far in detail, what degree of change in the specific gravity occurs in what type of cells is not known at all.

Under such circumstances, no one has proposed an idea of performing such cell fixation treatment before density gradient centrifugation, in which separation is achieved based strictly on the specific gravity.

However, the present inventors discovered that performing cell fixation treatment of cells contained in a blood-derived sample, followed by density gradient centrifugation of the resultant fixed cells, makes fixed rare cells almost securely contained in a layer containing a large amount of cells other than red blood cells, allowing improvement of the recovery of rare cells and reduction of the red blood cell contamination ratio, thereby completing the present invention.

That is, the method for removing red blood cells, which reflects one aspect of the present invention for achievement of at least one of the above-described objects, is a method in which red blood cells contained in a blood-derived sample are preliminarily removed in order to detect a rare cell(s) potentially contained in the blood-derived sample, which method comprises the steps of: (A) fixing cells contained in a blood-derived sample; and (B) subjecting the blood-derived sample obtained in the Step (A) to density gradient centrifugation to separate the sample into at least two layers, one of which contains a large amount of red blood cells and the other of which contains a large amount of cells other than red blood cells.

The centrifuge tube for blood collection, which reflects another aspect of the present invention, contains a cell fixative, an anticoagulant and a separation liquid for density gradient centrifugation preliminarily placed in the tube.

### EFFECT OF THE INVENTION

In cases where the method of removing red blood cells or the centrifuge tube for blood collection of the present invention is used for removing red blood cells, loss of rare cells hardly occurs and deterioration of cells such as shrinkage or swelling can be suppressed even with a blood-derived sample stored for a long period-for example, several days-after blood collection until the separation of red blood cells.

Further, in cases where the method of removing red blood cells or the centrifuge tube for blood collection of the present invention is used for removing red blood cells from a blood-derived sample, followed by spreading and observing the blood-derived sample from which red blood cells have been removed to thereby detect rare cells such as CTCs (potentially) contained in the blood-derived sample, reliability of the result of judgment on whether the rare cells are present in the blood-derived sample or not can be improved, and accuracy of detection of the rare cells from the blood-derived sample can be increased.

### BRIEF DESCRIPTION OF THE DRAWINGS

Fig. 1 is a schematic diagram showing a centrifuge tube (1) that has or has not been subjected to the density gradient centrifugation in Step (B) of the method of removing red blood cells of the present invention.
Fig. 2 is a graph showing changes in the leukocyte recovery over time observed in Example 2 and Comparative Example 2.
Fig. 3 is a graph showing: (a) changes in leukocytes over time; and (b) changes in the red blood cell contamination ratio over time; which were observed in Example 5.

### DESCRIPTION OF EMBODIMENTS

The method of removing red blood cells and the centrifuge tube for blood collection of the present invention are described below in detail.

### <Method of Removing Red Blood Cells>

The method of removing red blood cells of the present invention is a method for preliminarily removing red blood cells contained in a large amount in a blood-derived sample, for detection of a rare cell(s) potentially contained in the blood-derived sample. The method includes the Steps (A) and (B) below.

Cell fixation treatment step (A): a step of fixing cells contained in a blood-derived sample.

Density gradient centrifugation step (B): a step of subjecting the blood-derived sample after the fixation of cells to density gradient centrifugation to separate the sample into at least two layers, a "layer containing a large amount of red blood cells" and a "layer containing a large amount of cells other than red blood cells".

The "blood-derived sample" in the present invention means blood that has not been processed after collection, blood diluted with an appropriate diluent, blood whose coagulation was inhibited using an "anticoagulant" as described later, blood subjected to an appropriate treatment using a combination of agents such as a diluent and an anticoagulant, or the like.

In the cell fixation treatment step (A), not only cell fixation, but also inhibition of blood coagulation are preferably carried out, and, in a preferred mode, blood is reacted with an anticoagulant immediately after blood collection to inhibit blood coagulation, while a "cell fixative" is also added to fix blood cells.

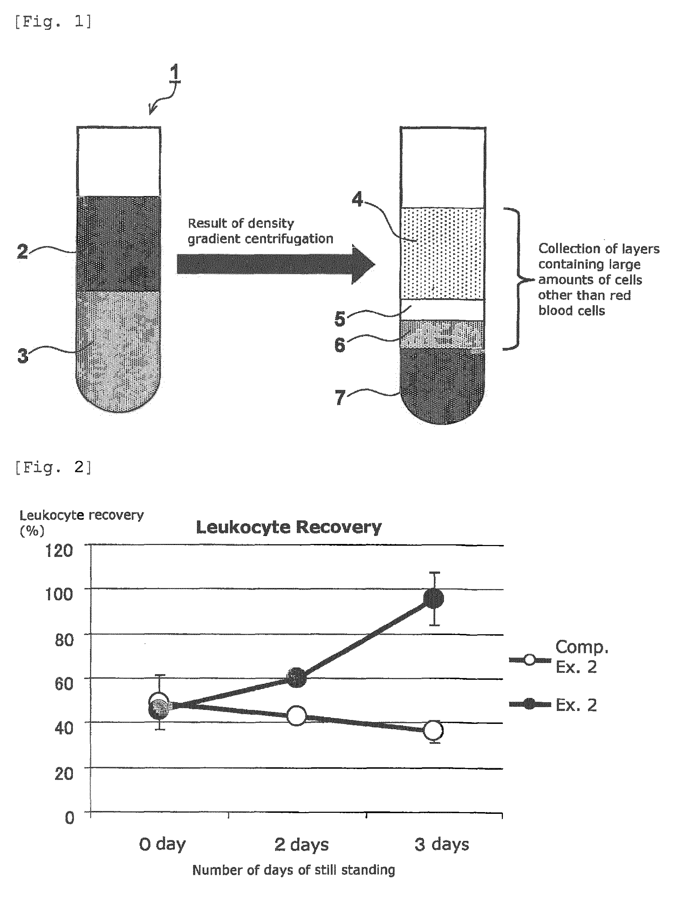

In one mode, the density gradient centrifugation step (B) can be divided, as shown in Fig. 1, into the steps of: overlaying the blood-derived sample after cell fixation (2) obtained in Step (A) on a separation liquid for density gradient centrifugation (3) preliminarily placed in a centrifuge tube (1) ; and subjecting this centrifuge tube (1) to density gradient centrifugation to separate the sample into a "layer containing a large amount of red blood cells" (7), "separation liquid layer" (6), "layer containing a large amount of mononuclear cells" (5) and "layer composed of plasma containing a large amount of platelets" (4).

The density gradient centrifugation step (B) is preferably followed by collection of all of the layers (4) to (6) in Fig. 1, which layers are not the "layer containing a large amount of red blood cells" (7) and correspond to the "layer containing a large amount of cells other than red blood cells".

In cases where the method of removing red blood cells of the present invention is used to remove red blood cells from a blood-derived sample before the spreading of cells and detection of the rare cells of interest, reliability of the result of judgment on whether the cells of interest are present in the blood-derived sample or not can be improved, and accuracy of detection of the cells of interest can be improved when the cells are present.

### [Rare Cells]

The "rare cells" that are potentially contained in the blood-derived sample used in the present invention is a general term for cells present in the body in a very small amount. Examples of the rare cells include circulating tumor cells (also referred to as blood-circulating cancer cells) [CTCs], vascular endothelial cells, vascular endothelial progenitor cells and various stem cells. These cells may be the cells of interest to be detected from the blood-derived sample obtained after carrying out the method for removing red blood cells of the present invention.

The rare cells in the present invention are especially preferably CTCs since CTCs can be used as an index for determination of prognosis of cancer, diagnosis of metastasis of cancer, and monitoring of a therapeutic effect on cancer.

### [Anticoagulant]

Anticoagulants are agents that prevent "blood coagulation", which is gelling of collected blood. Whether or not an anticoagulant is required depends on the use of the blood-derived sample. In cases where rare cells are collected as in the present invention, an anticoagulant is usually required.

Examples of commonly used "anticoagulants" include ethylenediaminetetraacetic acid [EDTA]; diethylenetriamine pentaacetic acid [DTPA]; 1,2-diaminocyclohexanetetraacetic acid [DCTA]; ethylenebis(oxyethylenenitrilo)tetraacetic acid [EGTA]; heparin species such as heparin, heparin sulfate and low molecular weight heparin; citric acid; and oxalic acid. For example, in cases where the number of leukocytes or percentage of lymphocytes is to be determined, EDTA is used as an anticoagulant. In cases where a subset of lymphocytes is to be investigated based on the CD4 count or the like, either heparin or EDTA may be used. In cases where blood for transfusion is to be collected, a liquid containing sodium citrate (e. g. , ACD [acid citrate dextrose] or CPD [citrate phosphate dextrose]) is used.

As the "anticoagulant" that can be used in the present invention, any of these conventionally known anticoagulants may be selected, but the anticoagulant in the present invention is not limited to these.

### [Cell Fixative]

In Step (A), that is, in the step of fixing cells contained in a blood-derived sample, a cell fixative is used to fix cells. The term "fixation" or "cell fixation" generally means a treatment carried out for the purpose of delaying autolysis or rotting of cells or a tissue, and of maintaining their morphology and antigenicity. In the present invention, such a treatment is effective for, for example, improvement of the recovery of rare cells and reduction of the red blood cell contamination ratio.

Examples of substances commonly used as cell fixatives include aldehydes, alcohols and heavy metal elements, but the cell fixative in the present invention is not limited to these. Examples of the cell fixative used in the present invention also include substances such as formaldehyde donors (which are also referred to as "formaldehyde donors" or "FA donors"), which do not have direct actions as cell fixatives but release cell fixatives such as formaldehyde by undergoing hydrolysis. Formaldehyde donors are especially preferred since they can release formaldehyde by hydrolysis from immediately after contacting with a blood-derived sample or blood, even after a long period of storage of the donors (under conditions where the donors do not undergo hydrolysis).

Aldehydes cause dissociation of existing binding modes (e.g., disulfide bonds and hydrogen bonds) of protein to form new bonds. Examples of these bonds include covalent bonds with amino group termini of lysine, arginine and the like and with aromatic active carbons such as tryptophan and thyroxine, and methylene cross-links formed by linking with other amino acid terminal residues. These new bonds can change higher-order structures of proteins, and can prevent further denaturation of free molecules that are not involved in such binding or cross-linking, by holding their polypeptide chains. Thus, aldehydes are cell fixatives that can stabilize protein structures, and can also suppress enzyme activities by gelling the cytoplast. A representative example of such aldehydes is formaldehyde [FA]. Glutaraldehyde and glyoxal are also included in such aldehydes.

Alcohols such as ethanol and methanol are cell fixatives that can denature proteins to cause precipitation.

Examples of the heavy metal elements include chrome [Cr], manganese [Mn] and zinc [Zn].

Examples of the formaldehyde donors include methylols of amines and amides; hydroxymethyl derivatives; diazolidinyl urea; imidazolidinyl urea; methenamine; and paraformaldehyde.

In the present invention, one of these may be used alone, or two or more of these may be used in combination. The cell fixative is not limited to these.

The amount of the cell fixative used in Step (A) may be arbitrarily set depending on the type of the fixative. The amount is controlled such that the concentration of the cell fixative is 0.01 to 50.0 vol% with respect to 100 vol% of blood contained in the blood-derived sample.

For example, in cases where an FA donor is used as the cell fixative, the FA donor is added such that its concentration is preferably 0.1 to 10.0 vol% with respect to 100 vol% of blood contained in the blood-derived sample. In cases where the concentration of the FA donor is within the range described above, FA is slowly released to allow maintenance of an FA concentration within the preferred concentration range described below for several days, which is preferred. This is because several days are usually required after blood collection in order to carry out the test.

For example, in cases where FA is used as the cell fixative, the FA is added such that its concentration is preferably 0.01 to 1.0 vol%, more preferably 0.02 to 0.2 vol%, with respect to 100 vol% of blood contained in the blood-derived sample. In cases where an alcohol is used, the alcohol is added such that the concentration of the cell fixative is preferably 1.0 to 50.0 vol%.

In cases where the cell fixative is used at a concentration within the range described above, the density gradient centrifugation in Step (B) can be carried out such that the layer containing a large amount of red blood cells hardly contains fixed rare cells. When the amount of the cell fixative is too large or too small, the leukocyte recovery and the red blood cell contamination ratio may be unsatisfactory in some cases.

The period of treatment in which the blood-derived sample is brought into contact with the cell fixative may be arbitrarily set, and it is preferred to secure a certain length of time such that the reaction proceeds after their mixing to achieve sufficient exertion of the action and effect of the present invention. Here, the present inventors consider that, for the achievement of sufficient exertion of the action and effect of the present invention, the balance between the concentration of the fixative and the treatment time tends to be important, and that the treatment time should be determined depending on the concentration of the fixative. For example, in typical cases such as the case using a Cyto-Chex (registered trademark) BCT as shown in the Examples described below, the treatment time is preferably 10 minutes to 7 days, in consideration of the degree of deterioration of the blood itself to be used as the sample.

### [Density Gradient Centrifugation]

The "density gradient centrifugation" carried out in Step (B) is a method mainly used in molecular biological experiments. In this method, a desired "separation liquid" is used to decrease, usually from the bottom to the top, the density (specific gravity) of a solution in a centrifuge tube, and a sample is centrifuged under such a condition to allow formation of a layer of a substance or cells of interest having a certain density (specific gravity) at the position having the corresponding density in the solution. By utilizing such properties, a subject is collected.

That is, since density gradient centrifugation is a method for separating a subject having a known density (specific gravity) from matters other than the subject, those skilled in the art do not normally use density gradient centrifugation for separating a subject having an unknown specific gravity.

The "separation liquid" used in the density gradient centrifugation is not limited as long as it is a separation liquid with a specific gravity whose osmotic pressure and pH can be adjusted such that destruction of cells does not occur. Examples of the separation liquid include Ficoll (registered trademark) and Percoll (registered trademark), which are commercially available, and sucrose solutions.

By carrying out density gradient centrifugation with the separation liquid having a specific gravity that is smaller than the specific gravity of red blood cells but larger than the specific gravity of leukocytes, the blood-derived sample can be separated into at least two layers, a "layer containing a large amount of red blood cells" and a "layer containing a large amount of cells other than red blood cells". By collecting the whole "layer containing a large amount of cells other than red blood cells", red blood cells can be eliminated almost completely from the blood-derived sample.

In cases where the specific gravity of the separation liquid is set to preferably not more than 1.113, more preferably not more than 1.085, the red blood cell contamination ratio in the whole collected "layer containing a large amount of cells other than red blood cells" can be suppressed to 2 to 6% or lower, which is preferred.

A combination of two or more separation liquids having different specific gravities (for example 1.077 and 1.119) may be used. In such cases, cells other than red blood cells (e.g., eosinophils, neutrophils, basophils, lymphocytes and mononuclear cells) can be further separated, and such cells, other than the rare cells to be detected, can therefore be further eliminated.

The specific gravities of major cellular components contained in the blood-derived sample typically have the relationship of: plasma < platelets < leukocytes < red blood cells. Although the specific gravity of CTCs varies depending on their type and properties, it is known to be almost the same as that of platelets or leukocytes, or even smaller than those of platelets and leukocytes.

### <Centrifuge Tube for Blood Collection>

In the centrifuge tube for blood collection of the present invention, the "cell fixative", the "anticoagulant" and the "separation liquid" for density gradient centrifugation are preliminarily placed. In a mode where these are preferably placed, the separation liquid is physically isolated from the cell fixative and the anticoagulant by a "porous barrier" and/or "thixotropic gel". In such cases, the cell fixative and the anticoagulant are preferably in the solid state (in the form of, for example, powder or granules). The cell fixative is especially preferably a formaldehyde donor.

The "porous barrier" is preferably made of an elastomer and in the form of a filter with penetrating holes each having a pore size of not less than the size that allows passing of a red blood cell (pore size of 5 to 8 µm) but not more than about 150 µm. The thickness of the porous barrier is not limited as long as it gives strength sufficient for preventing the barrier from being damaged by the mechanical force due to the density gradient centrifugation, and examples of the thickness include about 2 to 15 mm. In cases where only a porous barrier is used to isolate the separation liquid from the cell fixative and the anticoagulant, the cell fixative and the anticoagulant are preferably particles having sizes larger than the pore size of the porous barrier. The cell fixative and the anticoagulant are more preferably immobilized in the upper part of the centrifuge in the form of a film coating (s) so that they would not have contact with the separation liquid.

The "thixotropic gel" is a gel-like matter composed of a thixotropic substance selected from the group consisting of silicic acid, bentonite, hectorite, kaolin and alginate salt, and mixtures thereof. The thixotropic gel is fluidized by the action of the mechanical force due to density gradient centrifugation, but cured without the action of such a force.

Preferred examples of the porous barrier and the thixotropic gel for the centrifuge tube for blood collection of the present invention include those described in Japanese Unexamined Patent Application Publication (Translation of PCT Application) No. 2008-529541.

### EXAMPLES

The present invention is described below in more detail by way of Examples, but the present invention is not limited to these.

### [Example 1] Leukocyte Recovery and Red Blood Cell Contamination Ratio of Formaldehyde-treated Blood

As a blood collection tube, a Venoject (registered trademark) II vacuum blood collection tube "VP-DK053K" manufactured by Terumo was used. This blood collection tube preliminarily contained a predetermined amount of granules of ethylene diamine tetraacetic acid dipotassium salt [EDTA-2K]. It is presumed that this amount corresponded to an EDTA concentration of about 1.5 mg/mL with respect to the amount of blood collected. The capacity of the blood collection tube was 5 mL.

To 3 mL of the collected blood, 0.3 mL of a dilution of 20% neutral buffered formalin solution (manufactured by Wako Pure Chemical Industries, Ltd.) was added to carry out cell fixation. The mixture was then stirred by 10 times of inversion.

The "20% neutral buffered formalin solution" was prepared by mixing undiluted formalin solution (37% formaldehyde solution) with phosphate buffered saline (PBS) to adjust the concentration to about 7.4%, and the prepared solution was added to 3 mL of the blood in the blood collection tubes to a final formaldehyde [FA] concentration of 0.01 vol%, 0.02 vol%, 0.04 vol%, 0.08 vol% and 0.1 vol%, respectively.

Each blood collection tube was left to stand at room temperature for 3 days, and, on Day 3, was inverted again to allow the plasma component and the blood cell component to be mixed with each other, followed by diluting the content of the blood collection tube 2-fold with PBS solution supplemented with 0.5 wt% bovine serum albumin [BSA]. Four milliliters of the resulting dilution was overlaid on 3 mL of a separation liquid for density gradient centrifugation (Ficoll; specific gravity, 1.077) preliminarily placed in a centrifuge tube, and centrifugation was carried out at 400×g at room temperature for 40 minutes.

After the centrifugation, all layers other than the layer containing a large amount of red blood cells were collected and subjected to cell staining with a phycoerythrin [PE]-labeled anti-CD45 antibody and Hoechst 33342. Subsequently, 20 µL of the resulting sample was observed under the microscope using a hemacytometer. The total cell number was counted in the bright field, and the signals of PE and Hoechst 33342 observed by fluorescence excitation were counted as leukocytes.

Part of blood before the density gradient centrifugation was separately collected and similarly subjected to cell staining, followed by dilution with PBS solution as appropriate and counting of the number of leukocytes before centrifugation.

After correction with the dilution factor of the sample actually subjected to counting and the total amount of the sample, the total numbers of leukocytes present in 2 mL of the blood and in the centrifuged sample were calculated, and the ratio of the leukocyte count after the centrifugation to the leukocyte count before the centrifugation was calculated to determine the leukocyte recovery (%). In addition, a value given by subtracting the leukocyte count in the centrifuged sample from the total cell number was defined as the red blood cell count, and the ratio of the red cell count to the leukocyte count was calculated to determine the red blood cell contamination ratio. The results are shown in Table 1.

### [Comparative Example 1]

The leukocyte recovery and the red blood cell contamination ratio were determined in the same manner as in Example 1 except that PBS was used instead of the "20% neutral buffered formalin solution". The results are shown in Table 1.
[Table 1]

**Table 1: The leukocyte recovery and the red blood cell contamination ratio (red blood cell count / leukocyte count) on Day 3 after blood collection-Experiments at different fixation concentrations**

| | | Leukocyte recovery (%) | Red blood cell contamination ratio |
|---|---|---|---|
| Example 1 | 0.01% FA | 51. 6 | 1.8 |
| | 0.02% FA | 65.4 | 2.1 |
| | 0.04% FA | 73.1 | 1.0 |
| | 0.08% FA | 72.9 | 1.4 |
| | 0.1% FA | 69.1 | 0.5 |
| Comparative Example 1 | 0% FA | 36.1 | 2.6 |

### [Example 2] Leukocyte Recovery from Cyto-Chex (Registered Trademark)-treated Blood

To Cyto-Chex (registered trademark) BCTs (manufactured by Streck Innovations), which are commercially available as blood collection tubes preliminarily containing an anticoagulant and a cell fixative, 3 mL/tube of collected blood was placed.

As a result of analysis by the inventors, the cell fixative contained in the "Cyto-Chex (registered trademark) BCT" was expected to be formaldehyde donors such as diazolidinyl urea and imidazolidinyl urea. It is presumed that these donors exert the cell fixation action by sustained release of formaldehyde after mixing with blood. It is presumed that the anticoagulant contained in the "Cyto-Chex (registered trademark) BCT" was an EDTA solution. By using the Cyto-Chex (registered trademark) BCT as the blood collection tube, an effect of protecting surface antigens of leukocytes for a long time can be expected.

Subsequently, the blood collection tubes were inverted 10 times for mixing the samples.

Three such tubes, containing 3 mL of blood in Cyto-Chex (registered trademark), were provided and used for
a group in which the sample was left to stand for 10 minutes and then density gradient centrifugation was performed;
a group in which the sample was left to stand at room temperature for 2 days and then mixed once by inversion (such that the plasma component was mixed with the blood cell component), followed by performing density gradient centrifugation; and
a group in which the sample was left to stand at room temperature for 3 days and then mixed once by inversion, followed by performing density gradient centrifugation; respectively.

The content of each blood collection tube was diluted 2-fold with PBS solution supplemented with 0. 5% wt BSA. Four milliliters of the resulting dilution was overlaid on 3 mL of a separation liquid for density gradient centrifugation (Ficoll (registered trademark); specific gravity, 1.077) preliminarily placed in a centrifuge tube, and centrifugation was carried out at 400×g at room temperature for 40 minutes.

After the density gradient centrifugation, all layers other than the red blood cell layer were collected and subjected to cell staining with a PE-labeled anti-CD45 antibody and Hoechst. Subsequently, 20 µL of the resulting sample was observed under the microscope using a hemacytometer. The signals of PE and Hoechst 33342 observed by fluorescence excitation were counted as leukocytes. Further, part of the blood-derived sample before the density gradient centrifugation (after treatment with Cyto-Chex (registered trademark)) was separately collected and similarly subjected to cell staining, followed by dilution with PBS solution as appropriate and counting of the number of leukocytes before the density gradient centrifugation. After correction with the dilution factor of the sample actually subj ected to counting and the total amount of the sample, the total numbers of leukocytes present in 2 mL of the blood and in the centrifuged sample were calculated, and the ratio of the leukocyte count after the centrifugation to the leukocyte count before the centrifugation was calculated to determine the leukocyte recovery (%). The results are shown in Table 2 and Fig. 2.

### [Comparative Example 2]

The leukocyte recovery was determined in the same manner as in Example 2 except that "Venoject (registered trademark) II vacuum blood collection tube" (the same blood collection tube as the one used in Example 1, preliminarily containing a predetermined amount of EDTA-2K) was used instead of "Cyto-Chex (registered trademark) BCT". The results are shown in Table 2 and Fig. 2.
[Table 2]

**Table 2: Changes in the leukocyte recovery over time-Experiments with or without cell fixation**

| | | Leukocyte recovery (%) |
|---|---|---|
| Example 2 | Same day as blood collection | 48.5 |
| | Left to stand for 2 days | 59.8 |
| | Left to stand for 3 days | 95.9 |
| Comparative Example 2 | Same day as blood collection | 49.1 |
| | Left to stand for 2 days | 42.8 |
| | Left to stand for 3 days | 36.1 |

From Table 2, it was found that the leukocyte recovery after leaving the sample for 3 days in Example 2 was higher than the leukocyte recovery on the same day as the blood collection. The present inventors presume that this is due to slow release (sustained release) of formaldehyde from the cell fixative, which allowed the reaction with cells to proceed slowly.

### [Example 3] Comparison of Formaldehyde-treated Blood and Cyto-Chex (registered trademark)-treated Blood

A blood collection tube prepared in the same manner as Example 1 with addition of formaldehyde [FA] to 3 mL of blood at a final concentration of 0.08 vol%; and a blood collection tube prepared in the same manner as in Example 1 with addition of formaldehyde [FA] to 3 mL of blood at a final concentration of 0.16 vol%; were provided as a formaldehyde [FA]-treated group, and these tubes were treated in the same manner as in Example 1 except that the tubes were left to stand at room temperature for 1 day, instead of "leaving the tubes to stand at room temperature for 3 days". The leukocyte recovery was determined in the same manner as in Example 1.

A Cyto-Chex (registered trademark) BCT-treated group (hereinafter referred to as "Cyto-Chex (registered trademark) treatment") was provided in the same manner as in Example 2 except that the leaving and mixing were carried out by leaving the tube to stand for 1 day followed by one time of mixing by inversion, and the leukocyte recovery was determined.

The results are shown in Table 3.
[Table 3]

**Table 3: The leukocyte recovery (%) on Day 1 after blood collection-Use of different types and concentrations of fixatives**

| | | Leukocyte recovery (%) |
|---|---|---|
| Left to stand for 1 day | 0.08% FA | 103.5 |
| | 0.16% FA | 76.4 |
| | Cyto-Chex (registered trademark) treatment | 59.6 |
| (Reference) Left to stand for 3 days | Cyto-Chex (registered trademark) treatment | 95.9 |

It can be seen that, by treatment with formaldehyde [FA] at a sufficient final concentration, a leukocyte recovery (%) equivalent to that observed with Cyto-Chex (registered trademark) treatment can be obtained even after leaving the sample to stand for 1 day.

### [Example 4] Influence of Temperature during Still Standing on Leukocyte Recovery and Red Blood Cell Contamination Ratio

Different temperatures were employed during the 3 days of still standing in Example 2, and the leukocyte recovery and the red blood cell contamination ratio were determined. That is, an operation was carried out in the same manner as in Example 2 except that, in the cell fixation using "Cyto-Chex (registered trademark) BCT", the temperature at which each sample was left to stand for 3 days was selected from the following 4 different temperatures: 4°C, 20°C, 29°C and 37°C. The leukocyte recovery and the red blood cell contamination ratio were determined for each sample.

The results are shown in Table 4.
[Table 4]

**Table 4: The leukocyte recovery and the red blood cell contamination ratio on Day 3 after blood collection-Still standing at different temperatures**

| Still-standing temperature | Leukocyte recovery (%) | Red blood cell contamination ratio |
|---|---|---|
| 4 °C | 62.7 | 0.02 |
| 20 °C | 107.6 | 0.04 |
| 29 °C | 74.1 | 0.13 |
| 37 °C | 33.9 | 0.37 |

It can be seen that employment of the room temperature (20°C) gave especially good results in terms of the leukocyte recovery and the red blood cell contamination ratio.

### [Example 5] Changes in Leukocyte Recovery and Red Blood Cell Contamination Ratio over Time at 4°C and 20°C

Changes in the leukocyte recovery and the red blood cell contamination ratio were monitored by the same method as in Example 2 for 5 days after blood collection, wherein the samples were stored at 4°C or 20°C, which are temperatures assumed to be suitable for long-term storage in Example 4. That is, an operation was carried out in the same manner as in Example 2 except that, in the cell fixation using "Cyto-Chex (registered trademark) BCT", the temperature at which each sample was left to stand was selected from the following 2 different temperatures: 4°C and 20°C, and the period during which each sample was left to stand was selected from the following 3 different periods: 10 minutes (0 day), 3 days (3 days) and 5 days (5days). The leukocyte recovery and the red blood cell contamination ratio were calculated for each sample.

Here, samples treated without cell fixation, similarly to Comparative Example 2, were also subjected to monitoring of changes in the leukocyte recovery and the red blood cell contamination ratio over time at 20°C.

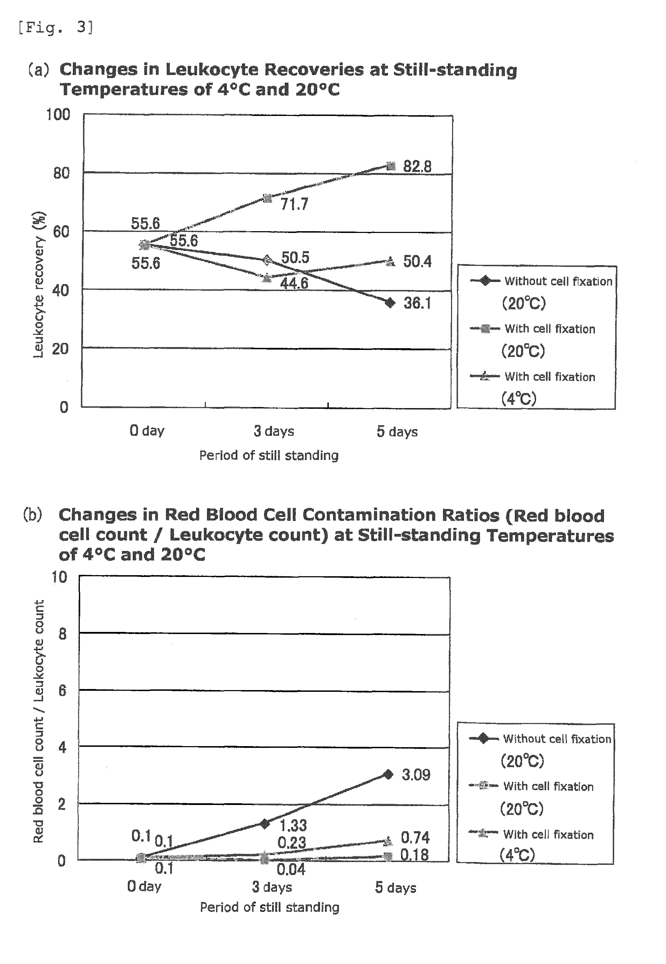

The results are shown in Fig. 3.

### DESCRIPTION OF SYMBOLS

- 1: Centrifuge tube
- 2: Blood-derived sample after cell fixation
- 3: Separation liquid
- 4: Layer composed of plasma containing a large amount of platelets
- 5: Layer containing a large amount of mononuclear cells
- 6: Separation liquid layer
- 7: Layer containing a large amount of red blood cells

## Claims

1. A method of removing red blood cells for preliminarily removing red blood cells contained in a blood-derived sample for detection of a rare cell(s) potentially contained in said blood-derived sample, said method comprising the steps of:
(A) fixing cells contained in a blood-derived sample; and
(B) subjecting the blood-derived sample obtained in the Step (A) to density gradient centrifugation to separate said sample into at least two layers, one of which contains a large amount of red blood cells and the other of which contains a large amount of cells other than red blood cells.

2. The method of removing red blood cells according to claim 1, wherein, in the Step (A), a cell fixative is added such that the concentration of said cell fixative is not less than 0.01 vol% and not more than 50.0 vol% with respect to 100 vol% of blood contained in said blood-derived sample.

3. The method of removing red blood cells according to claim 1, wherein, in cases where a formaldehyde donor is used as a cell fixative for fixing the cells in the Step (A), said formaldehyde donor is added such that the concentration of said formaldehyde donor is not less than 0.1 vol% and not more than 10.0 vol% with respect to 100 vol% of blood contained in said blood-derived sample.

4. The method of removing red blood cells according to claim 1, wherein, in cases where formaldehyde is used as a cell fixative for fixing the cells in the Step (A), said formaldehyde is added such that the concentration of said formaldehyde is not less than 0.01 vol% and not more than 1.0 vol% with respect to 100 vol% of blood contained in said blood-derived sample.

5. The method of removing red blood cells according to claim 1, wherein, in cases where an alcohol is used for fixing the cells in the Step (A), said cell fixative is added such that the concentration of said cell fixative is not less than 1.0 vol% and not more than 50.0 vol% with respect to 100 vol% of blood contained in said blood-derived sample.

6. The method of removing red blood cells according to any one of claims 1 to 5, wherein the period of cell fixation in the Step (A) is not less than 10 minutes and not more than 7 days.

7. The method of removing red blood cells according to any one of claims 1 to 6, wherein said cell fixation in the Step (A) is carried out together with inhibition of blood coagulation.

8. The method of removing red blood cells according to any one of claims 1 to 7, wherein, in the Step (B), the specific gravity of a separation liquid used for the density gradient centrifugation is not more than 1.113.

9. The method of removing red blood cells according to any one of claims 1 to 7, wherein, in the Step (B), the specific gravity of a separation liquid used for the density gradient centrifugation is not more than 1.085.

10. The method of removing red blood cells according to any one of claims 1 to 9, wherein the Step (B) is followed by collection of all layers other than said layer containing a large amount of red blood cells.

11. A centrifuge tube for blood collection wherein a cell fixative, an anticoagulant and a separation liquid for density gradient centrifugation are preliminarily placed.
